# EUROPÄISCHE PATENTSCHRIFT

(11) **EP 1 251 896 B1**
(45) Veröffentlichungstag und Bekanntmachung des Hinweises auf die Patenterteilung: **01.10.2003**
(21) Anmeldenummer: 01911584.9
(22) Anmeldetag: 02.02.2001
(51) Int. Cl.: A61M 15/00

(54) **INHALATIONSVERNEBLER**
INHALATION NEBULIZER
ATOMISEUR POUR INHALATION

(30) Priorität: 02.02.2000 DE 10004465
(43) Veröffentlichungstag der Anmeldung: 30.10.2002
(73) Patentinhaber: PARI GmbH Spezialisten für effektive Inhalation, 82319 Starnberg (DE)
(72) Erfinder: SOMMER, Erik, 15566 Schöneiche bei Berlin (DE)
(74) Vertreter: HOFFMANN - EITLE
(86) Internationale Anmeldenummer: EP0101156
(87) Internationale Veröffentlichungsnummer: WO01056639

(56) Entgegenhaltungen:
- EP-A- 0 170 715
- EP-A- 0 642 992
- WO-A-96/04123
- WO-A-96/32345
- WO-A-99/42154
- US-A- 3 354 022

## Beschreibung

Die Erfindung betrifft Inhalationsvernebler mit einem Flüssigkeitsreservoir für eine zu vernebelnde Flüssigkeit.

Mit Inhalationsverneblern werden therapeutisch wirksame oder medikamenthaltige Flüssigkeiten mit Hilfe eines Aerosolerzeugers zu einem Aerosol vernebelt, das regelmäßig aus lungengängigen Partikeln besteht. Das erzeugte Aerosol wird einem Patienten im Rahmen einer Inhalationstherapie zum Einatmen dargeboten, wodurch die therapeutisch wirksame Flüssigkeit oder das Medikament in die Atemwege des Patienten gelangen.

Inhalationsvernebler dieser Art sind in unterschiedlichen Ausgestaltungen bekannt. So offenbart beispielsweise EP 0 170 715 A1 einen Inhalationsvernebler, bei dem u.a. flüssige Stoffe mittels eines Druckgasstromes vernebelt werden. Eine Düse ist als Aerosolerzeuger in einem Verneblungsraum des Inhalationsverneblers angeordnet und besitzt zwei neben einem Druckgaskanal angeordnete Ansaugkanäle. Wenn Druckluft durch den Druckgaskanal strömt, wird die zu zerstäubende Flüssigkeit durch die Ansaugkanäle angesaugt. Dazu ragen die Ansaugkanäle in einen Behälter, in dem die zu zerstäubende Flüssigkeit bevorratet wird. Ein nach dem gleichen Prinzip arbeitender Inhalationsvernebler ist aus EP 0 694 314 A1 bekannt, bei dem aber zur Verbesserung der Dosisgenauigkeit ein Behältereinsatz für das Zerstäubungsgut vorgesehen ist. Diese Ausgestaltung wird vorgeschlagen vor dem Hintergrund der Anforderung, dass bei medizinischen Anwendungen die genaue Dosierung des zu verabreichenden Medikaments besonderes wichtig ist. Dieser Anforderung wird bei den bekannten Inhalationsverneblern dadurch Rechnung getragen, dass genau definierte Mengen des Medikaments zur Zerstäubung bereitgestellt werden.

Die zur Verneblung bereitgestellte Flüssigkeitsmenge ist bei Inhalationsverneblern der hier in Rede stehenden Art nie gleich der tatsächlich vernebelten Flüssigkeitsmenge, so dass die dem Patienten zur Inhalation dargebotene Medikamentenmenge stets kleiner als die in den Inhalationsvernebler eingefüllte Medikamentenmenge ist. Insbesondere bei medikamenthaltigen Flüssigkeiten ist zu beobachten, dass die Konzentration des Medikaments in der im Vernebler verbleibenden Flüssigkeit im Verlauf der Inhalationsbehandlung ansteigt.

Ausgehend von diesem Stand der Technik besteht das der Erfindung zugrunde liegende Problem darin, die bekannten Inhalationsvernebler derart weiterzubilden, dass eine höhere Dosisgenauigkeit erreicht wird, d.h. dass die mit dem Inhalationsvernebler vernebelte Flüssigkeit und die dem Patienten verabreichte wirksame Dosis genauer bestimmbar ist.

Dieses Problem wird gelöst durch einen Inhalationsvernebler mit einem Flüssigkeitsreservoir für eine zu vernebelnde, therapeutisch wirksame oder medikamenthaltige Flüssigkeit, bei dem die der Flüssigkeit zugewandte Oberfläche des Flüssigkeitsreservoirs eine künstliche Oberflächenstruktur aus Erhebungen und Vertiefungen aufweist, wobei zumindest die Erhebungen der Oberflächenstruktur aus hydrophoben Materialien oder dauerhaft hydrophobierten Materialien bestehen und die Erhebungen nicht durch die Flüssigkeit, durch Wasser oder durch Wasser mit Detergenzien ablösbar sind.

Durch die erfindungsgemäße Gestaltung des Inhalationsverneblers wird erreicht, dass die Flüssigkeit oder das in ihr enthaltene Medikament nicht an der Oberfläche des Flüssigkeitsreservoirs anhaftet. Dadurch wird Flüssigkeitsverlust aufgrund von Benetzung der Oberflächen vermieden. Außerdem wird bei entsprechend gestalteten Verneblern die Rückführung der Flüssigkeit in den Ansaugbereich des Aerosolerzeugers unterstützt. In ganz erheblichem Masse wird die Dosisgenauigkeit in den Fällen erhöht, in dem die in der Flüssigkeit enthaltenen Medikamentenpartikel stärker an einer nicht erfindungsgemäß ausgestalteten Oberfläche anhaften als die Flüssigkeit selbst. Durch die erfindungsgemäße Oberflächenstruktur wird erreicht, dass das Medikament stets in der Flüssigkeit verbleibt und damit mit größerer Zuverlässigkeit vernebelt werden.

In einer bevorzugten Ausgestaltung beträgt der Abstand zwischen den Erhebungen der erfindungsgemäßen Oberflächenstruktur im Bereich von 1 bis 300 µm. Die Höhe der Erhebungen liegt im Bereich von 1 bis 100 µm.

Vorteilhaft ist ferner die Verwendung eines Polymers als hydrophobes Material.

Um auszuschließen, dass andere Bereiche aufgrund von Anhaftung der Flüssigkeit bzw. des Medikaments zu einer undefinierten Verringerung der zu vernebelnden Flüssigkeitsmenge oder der Medikamentenmenge beitragen, ist in einer weiteren Ausgestaltung der Erfindung vorgesehen, dass die dem Verneblungsraum zugewandte Oberfläche des Inhalationsverneblergehäuses ebenfalls die zuvor beschriebene künstliche Oberflächenstruktur besitzt. In einer weiteren Ausgestaltung wird diese Oberflächenstruktur auch auf den im Verneblungsraum liegenden Oberflächen eines Zuluftkamins vorgesehen. Letztlich können alle dem Verneblungsraum zugewandten Oberflächen des Inhalationsverneblers mit der erfindungsgemäßen Oberflächenstruktur ausgestattet werden, um einen Flüssigkeits- bzw. Medikamentenverlust aufgrund von Benetzung und Anhaftung optimal zu vermeiden. Jedoch ist anzumerken, dass davon ausgegangen werden kann, dass die erfindungsgemäße Ausgestaltung der Oberfläche des Flüssigkeitsreservoirs den größten Beitrag bei der Erhöhung der Dosisgenauigkeit leistet.

Die erfindungsgemäß vorzusehende Oberflächenstruktur lässt sich auf unterschiedliche Weise herstellen, wie beispielsweise in EP 0 772 514 A1 für selbstreinigende Oberflächen beschrieben ist. Dabei ist überraschend, dass eine Oberflächenstruktur, die bei großen Objekten wie Autokarosserien, Gebäudefassaden und ähnlichem zum Zwecke der Reinhaltung vorgeschlagen wird, im Bereich der Inhalationsvernebler zur Erhöhung der Dosisgenauigkeit angewendet werden kann.

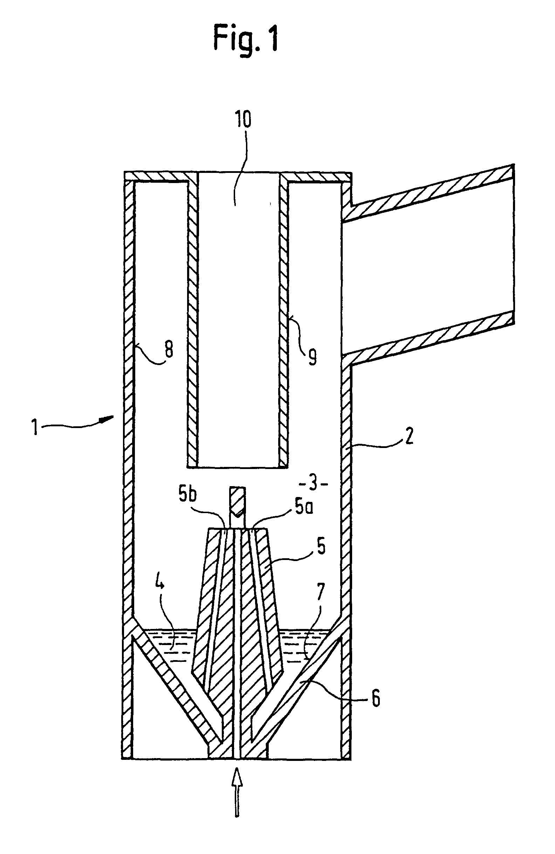

Im folgenden wird die Erfindung anhand eines Ausführungsbeispiels unter Bezugnahme auf die beigefügte Figur 1 genauer beschrieben.

Der in der Figur 1 gezeigte Inhalationsvernebler 1 besteht aus einem Gehäuse 2, das einen Verneblungsraum 3 umschließt, in den hinein die zu vernebelnde Flüssigkeit 4 mit Hilfe eines Aerosolerzeugers 5 zerstäubt wird. Die Flüssigkeit 4 befindet sich während des Gebrauchs in einem Flüssigkeitsreservoir 6, das so ausgebildet ist, dass sich die Flüssigkeit am Fuß des Aerosolerzeugers sammelt und über die Ansaugkanäle 5a und 5b des Aerosolerzeugers 5 angesaugt werden kann.

Bei dem in diesem Ausführungsbeispiel beschriebenen Aerosolerzeuger handelt es sich um eine nach den Venturi-Prinzip arbeitende Zerstäuberdüse. Die Erfindung ist aber nicht auf Inhalationsvernebler mit dieser Aerosolerzeugerart beschränkt.

Erfindungsgemäß ist die Oberfläche 7 des Flüssigkeitsreservoirs mit einer künstlichen Oberflächenstruktur aus Erhebungen und Vertiefungen ausgestattet. Zumindest die Erhebungen der Oberflächenstruktur bestehen aus hydrophoben Materialien, vorzugsweise Polymeren, oder aus dauerhaft hydrophobierten Materialien. Der Abstand zwischen den Erhebungen liegt im Bereich von 1 bis 300 µm, vorzugsweise von 5 bis 200 µm. Die Höhe der Erhebungen liegt im Bereich von 1 bis 100 µm. Um den erfindungsgemäßen Effekt zu erhalten, sind die Erhebungen weder durch die Flüssigkeit, noch durch Wasser oder durch Wasser mit Detergenzien ablösbar.

Durch die erfindungsgemäß gestaltete Oberfläche 7 wird sichergestellt, dass keine Benetzung stattfindet und somit die Flüssigkeit und/oder das Medikament in verstärktem Umfang zum Düsenfuß und damit zu den Ansaugkanälen 5a und 5b des Aerosolerzeugers 5 geführt werden.

Bei dem in der Figur gezeigten Inhalationsvernebler ist ferner die innere Oberfläche 8 des Inhalationsverneblers 1, also die Fläche, die den Verneblungsraum 3 im wesentlichen umschließt, mit einer künstlichen Oberflächenstruktur aus Erhebungen und Vertiefungen ausgestattet. Die Oberflächenstruktur entspricht der Oberflächenstruktur des Flüssigkeitsreservoirs, muss aber in der konkreten Ausgestaltung nicht identisch sein. So kann z.B. der Abstand der Erhebungen im Bereich des Flüssigkeitsreservoirs kleiner, im Bereich des Verneblungsraumes größer sein.

In ähnlicher Weise kann die erfindungsgemäße Oberflächenstruktur auf der Oberfläche 9 des Zuluftkamins 10 vorgesehen sein, wobei auch hier die konkrete Gestaltung nicht mit der Oberfläche 7 des Flüssigkeitsreservoirs 6 oder der Oberfläche 8 des Verneblergehäuses 2 übereinstimmen muss.

## Patentansprüche

1. Inhalationsvernebler mit einem Flüssigkeitsreservoir für eine zu vernebelnde, therapeutisch wirksame oder medikamenthaltige Flüssigkeit
**dadurch gekennzeichnet, dass**
die der Flüssigkeit zugewandte Oberfläche des Flüssigkeitsreservoirs eine künstliche Oberflächenstruktur aus Erhebungen und Vertiefungen aufweist, dass zumindest die Erhebungen der Oberflächenstruktur aus hydrophoben Materialien oder dauerhaft hydrophobierten Materialien bestehen und die Erhebungen nicht durch die Flüssigkeit durch Wasser oder durch Wasser mit Detergenzien ablösbar sind.

2. Inhalationsvernebler nach Anspruch 1,
**dadurch gekennzeichnet, dass**
der Abstand zwischen den Erhebungen im Bereich von 1 bis 200 µm und die Höhe der Erhebungen von 1 bis 100 µm liegen.

3. Inhalationsvernebler nach Anspruch 1 oder 2,
**dadurch gekennzeichnet, dass**
das hydrophobe Material ein Polymer ist.

4. Inhalationsvernebler nach einem der Ansprüche 1, 2 oder 3, **dadurch gekennzeichnet, dass**
in dem Inhalationsvernebler ein Verneblungsraum vorgesehen ist, in dem die Flüssigkeit vernebelt wird, und dass die dem Verneblungsraum zugewandte Oberfläche des Inhalationsverneblers eine künstliche Oberflächenstruktur aus Erhebungen und Vertiefungen aufweist, dass zumindest die Erhebungen der Oberflächenstruktur aus hydrophoben Materialien oder dauerhaft hydrophobierten Materialien bestehen und die Erhebungen nicht durch die Flüssigkeit, durch Wasser oder durch Wasser mit Detergenzien ablösbar sind.

5. Inhalationsvernebler nach einem der Ansprüche 1 bis 4, **dadurch gekennzeichnet, dass** das Flüssigkeitsreservoir derart gestaltet ist, dass eine Rückführung der Flüssigkeit in den Ansaugbereich eines in Flüssigkeitsreservoir angeordneten Aerosolerzeugers erfolgt.

6. Inhalationsvernebler nach Anspruch 5, **dadurch gekennzeichnet, dass** in dem Verneblungsraum ein Zuluftkamin angeordnet ist und dass die in dem Verneblungsraum liegenden Oberflächen des Zuluftkamins eine künstliche Oberflächenstruktur aus Erhebungen und Vertiefungen aufweist, dass zumindest die Erhebungen der Oberflächenstruktur aus hydrophoben Materialien oder dauerhaft hydrophobierten Materialien bestehen und die Erhebungen nicht durch die Flüssigkeit, durch Wasser oder durch Wasser mit Detergenzien ablösbar sind.

## Claims

1. Inhalation nebuliser having a liquid reservoir for a therapeutically effective or medicament-containing liquid to be nebulised, **characterised in that** the surface of the liquid reservoir facing the liquid has an artificial surface structure of projections and depressions, **in that** at least the projections of the surface structure consist of hydrophobic materials or materials rendered permanently hydrophobic and the projections cannot be removed by the liquid, by water or by water with detergents.

2. Inhalation nebuliser according to claim 1, **characterised in that** the distance between the projections lies in the range from 1 to 200 µm and the height of the projections from 1 to 100 µm.

3. Inhalation nebuliser according to claim 1 or 2, **characterised in that** the hydrophobic material is a polymer.

4. Inhalation nebuliser according to one of claims 1, 2 or 3, **characterised in that** in the inhalation nebuliser, a nebulising chamber is provided in which the liquid is nebulised, and **in that** the surface of the inhalation nebuliser facing the nebulising chamber has an artificial surface structure of projections and depressions, **in that** at least the projections of the surface structure consist of hydrophobic materials or materials rendered permanently hydrophobic and the projections cannot be removed by the liquid, by water or by water with detergents.

5. Inhalation nebuliser according to one of claims 1 to 4, **characterised in that** the liquid reservoir is designed such that return of the liquid to the intake region of an aerosol generator arranged in liquid reservoir is effected.

6. Inhalation nebuliser according to claim 5, **characterised in that** a supply air flue is arranged in the nebulising chamber and **in that** the surfaces of the supply air flue lying in the nebulising chamber has an artificial surface structure of projections and depressions, **in that** at least the projections of the surface structure consist of hydrophobic materials or materials rendered permanently hydrophobic and the projections cannot be removed by the liquid, by water or by water with detergents.

## Revendications

1. Nébuliseur à inhalation avec un réservoir à liquide pour nébuliser un liquide à action thérapeutique ou contenant un médicament,
**caractérisé en ce que**
la surface du réservoir à liquide orientée vers ledit liquide présente une structure superficielle qui comprend des saillies et des cavités, **en ce que** au moins les saillies de ladite structure superficielles sont en matières hydrophobes ou en matières rendues durablement hydrophobes, et **en ce que** lesdites saillies ne peuvent pas se dissoudre sous l'action du liquide, d'eau ou d'eau additionnée de détergents.

2. Nébuliseur à inhalation selon la revendication 1,
**caractérisé en ce que**
la distance entre les saillies est de l'ordre de 1 à 200 µm, et la hauteur desdites saillies de 1 à 100 µm.

3. Nébuliseur à inhalation selon la revendication 1 ou 2, **caractérisé en ce que**
la matière hydrophobe est un polymère.

4. Nébuliseur à inhalation selon les revendications 1, 2 ou 3, **caractérisé en ce que**,
dans le nébuliseur à inhalation, une chambre de nébulisation est prévue, dans laquelle le liquide est nébulisé, et que la surface du nébuliseur à inhalation orientée vers la chambre de nébulisation présente une structure superficielle, artificielle comprenant des saillies et des cavités, que les saillies de la structure superficielle, elles au moins, sont en matières hydrophobes ou rendues durablement hydrophobes et ne peuvent pas se dissoudre sous l'action du liquide, d'eau ou d'eau additionnée de détergents.

5. Nébuliseur à inhalation selon la revendication 1 à 4, **caractérisé en ce que** le réservoir à liquide est conçu de sorte que le liquide soit reconduit dans la zone d'aspiration d'un générateur aérosol qui est disposé dans ledit réservoir à liquide.

6. Nébuliseur à inhalation selon la revendication 5, **caractérisé en ce que**, dans la chambre de nébulisation, une cheminé de prise d'air est disposée et que les surfaces de ladite cheminée de prise d'air situées dans la chambre de nébulisation présentent une structure superficielle, artificielle comprenant des saillies et des cavités, que les saillies de la -structure superficielle, elles au moins, sont en matières hydrophobes ou rendues durablement hydrophobes et ne peuvent pas se dissoudre sous l'action du liquide, d'eau ou d'eau additionnée de détergents.
